(19) Europäisches Patentamt
European Patent Office
Office européen des brevets

(11) Veröffentlichungsnummer: **0 276 687**
**A2**

(12) # EUROPÄISCHE PATENTANMELDUNG

(21) Anmeldenummer: **88100393.3**

(22) Anmeldetag: **13.01.88**

(51) Int. Cl.⁴: **C07H 19/01** , A01N 43/90

(30) Priorität: **26.01.87 DE 3702175**

(43) Veröffentlichungstag der Anmeldung:
**03.08.88 Patentblatt 88/31**

(84) Benannte Vertragsstaaten:
**AT BE CH DE ES FR GB GR IT LI LU NL SE**

(71) Anmelder: **Max-Planck-Gesellschaft zur Förderung der Wissenschaften e.V.**
**Bunsenstrasse 10**
**D-3400 Göttingen(DE)**

(72) Erfinder: **Rembold, Heinz, Prof. Dr.**
**Wolfratshauserstrasse 68a**
**D-8000 München 70(DE)**
Erfinder: **Forster, Hans**
**Hinterbärenbadstrasse 76**
**D-8000 München 70(DE)**

(74) Vertreter: **Huber, Bernhard, Dipl.-Chem. et al**
**Möhlstrasse 22 Postfach 860 820**
**D-8000 München 86(DE)**

(54) **Azadirachtin-artige Verbindungen und ihre Verwendung in Insektenvernichtungsmitteln.**

(57) Ein neues Insektenvernichtungsmittel enthält als Wirkstoff mindestens eine Verbindung, die als Strukturelement ein Azadirachtingerüst der allgemeinen Formel

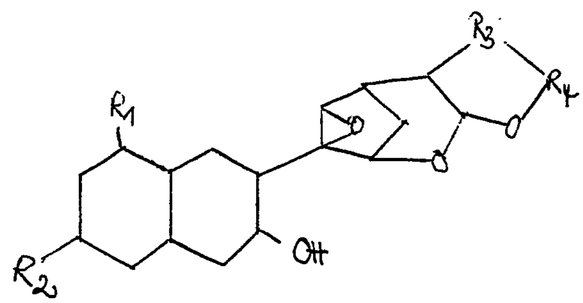

wobei

$R_1$ = OH oder ein Acylrest einer geradkettigen oder verzweigten gesättigten oder wenn $R_2$ eine andere Acylgruppe als Acetyl, oder/und $R_3$ sowie $R_4$ jeweils C mit H oder einer Alkoxygruppe als Ligand sind, ungesättigten Monocarbonsäure mit bis zu 10 C-Atomen,

$R_2$ = OH oder ein Acylrest einer geradkettigen oder verzweigten gesättigten oder, wenn $R_1$ eine Acylgruppe oder/und $R_3$ sowie $R_4$ jeweils C mit H oder einer Alkoxygruppe als Ligand sind, ungesättigten Monocarbonsäure mit bis zu 10 C-Atomen,

$R_3$ und $R_4$ jeweils C mit H oder einer Alkoxygruppe mit bis zu 6 C-Atomen als Liganden oder einer zusätzlichen Bindung zu $R_4$ bzw. $R_3$ bedeutet.

Die Wirkstoffe sind größtenteils neu. Zur Herstellung des Mittels verseift man einen Extrakt aus Kernen von Nüssen des Neembaumes in schwach basischer Lösung bei Raumtemperatur.

EP 0 276 687 A2

## Azadirachtin-artige Verbindungen und ihre Verwendung in Insektenvernichtungsmitteln

Die Erfindung betrifft neue azadirachtinartige Verbindungen aus den Kernen von Nüssen des Neembaumes, die Herstellung von Insektenvernichtungsmitteln aus dem Extrakt von Neemnüssen, sowie Insektenvernichtungsmittel, die die azadirachtinartigen Verbindungen enthalten.

Seit etwa 100 Jahren werden Insekten mit Insektiziden bekämpft. Die bekanntesten Insektizide der neuen Generation sind die chlorierten Kohlenwasserstoffe, wie DDT, Aldrin und Lindan. Diese Stoffe sind auch für den Menschen giftig. Ferner verursachen sie große ökologische Schäden, weil sie nur langsam abgebaut werden. Viele Insektenarten haben in den letzten Jahren Resistenzen gegen toxische Insektizide entwickelt und können dadurch mit den bisher verwendeten Stoffen nicht mehr angegriffen werden. Etwa 400 Schadinsektenarten sind bis heute gegen die herkömmlichen Insektizide mehr oder weniger resistent.

Diese Resistenzbildung fordert die Entwicklung von neuen Stoffen heraus, mit welchen die Insekten noch angreifbar sind. Die neuen Insektizide müssen ökologisch unbedenklich und für andere Tierarten ungiftig sein. Seit langem versucht man, vor allem in den Entwicklungsländern, Insektizide aus dem Pflanzenreich zu gewinnen. Schon seit 1690 wurden Tabakextrakte, deren aktives Prinzip das Nikotin ist, in England zum Töten von saugenden Insekten benutzt. Auch die Gruppe der Rotenoide, welche in den Wurzeln von Derris elliptica vorkommt, ist gegen viele Insekten wirksam, jedoch beinahe harmlos für Warmblütler.

Zu den modernsten Insektiziden, die zur Zeit eingesetzt werden können, zählt das Pyrethrum aus Chrysanthemum cinerariaefolium bzw. die Pyrethroide.

Eine weitere Pflanze, aus der ein Insektizid entwickelt werden könnte, ist der Neembaum, der in Indien und Ostafrika weit verbreitet ist. Dieser Neembaum wird nur von wenigen Insekten befallen. Dies hat das Interesse von Insektenforschern geweckt. Viele Inhaltstoffe dieses Baumes wurden isoliert. Einige davon haben frass-oder wachstumshemmende Wirkung auf Insekten. Die wirksamste dieser Verbindungen ist das Azadirachtin.

Insektenwachstumshemmer sind Substanzen, die in Insekten das Wachstum oder bestimmte Entwicklungsprozesse ändern. Die meisten der heute bekannten Insektizide wirken auf diese Art, z. B. DDT, Aldrin, Pyrethrum. Diese Substanzen sind für Insekten spezifisch, da bei Insekten die meisten Wachstums-und Entwicklungsvorgänge anders als bei anderen Organismen ablaufen. So werden die Larven-Larven-, Larven-Puppen-und Puppen-Adult-Häutungen unter anderem durch das Häutungshormon Ecdyson gesteuert. Die Art der Häutung wird durch die Juvenilhormon(JH)-Konzentration bestimmt. Die meisten der bis jetzt bekannten Insektenwachstumshemmer wirken gegen das Zusammenspiel der beiden Insektenhormone Ecdyson und Juvenilhormon.

Bei fast allen Insekten gibt es Unterschiede in der Populationsdichte, die von der Jahreszeit oder vom landwirtschaftlichen Zustand des befallenen Gebietes abhängen. Mit Wachstumshemmern ist es deshalb möglich, den Populationsanstieg der nächsten Generation zu verhindern.

Es gibt verschiedene Typen von Insektenwachstumshemmern:

a) Juvenilhormone

Schädlingskontrolle mit Regulatoren (z. B. Hormonen) kann nur dann erfolgreich sein, wenn es im Leben des Insekts eine Zeit gibt, in der die Abwesenheit dieser Substanz wichtig ist. Dies ist im Fall von JH gegeben. Im großen Rahmen kann JH aber nicht eingesetzt werden, da die Struktur zu kompliziert ist, um zu wirtschaftlich tragbaren Kosten produziert werden zu können. Außerdem sind Juvenilhormone labile Verbindungen und auch deshalb ist eine Anwendung auf dem Feld nicht möglich.

b) Häutungshormone

Diese Hormone sind im Leben des Insekts nötig, um die Häutung zum nächsten Larvenstadion zu veranlassen. Die Häutungshormone Ecdyson und 20-Hydroxy-Ecdyson haben aber ein Steroidgerüst, das nur schwer und teuer zu synthetisieren ist. Außerdem sind viele Hormone des Menschen dem Ecdysonstrukturell ähnlich. Ein großflächig angewandtes Ecdysonanaloges könnte daher auch für den Menschen - schädlich sein.

c) Hormonantagonisten

Eine Inhibierung von Hormonaktivitäten ist schädlich für das sich entwickelnde Insekt. Das bekannteste Antijuvenilhormon ist das Precocen. Es verursacht bei einigen Insekten eine vorzeitige Metamorphose und verhindert die Eientwicklung bei den erwachsenen Tieren. Andere Hormonantagonisten sind z. B. Piperonyl-Butoxid sowie bestimmte Azasteroide. Diese Stoffe unterbinden die Entwicklung und Metamorphose der Insekten, manche dieser Stoffe sind jedoch auch schädlich für den Menschen.

In Indien, Togo, Bangladesh und anderen Ländern wurden Neemformulierungen auf Feldern getestet und die Ergebnisse in "Natural pesticides from the Neem tree and other tropical plants", 1984, ed. H. Schmutterer, K.R.S. Ascher, GTZ, Eschborn, S. 435 (M. Dreyer), S. 263 (B. N. Islam) und S. 565 (S. Ahmed et al.) veröffentlicht. Es zeigte sich, daß der Insektenfraß reduziert und der Ernteertrag gesteigert wurde. Diese Art der Insektenkontrolle kann jedoch nur auf begrenztem Raum angewandt werden. Der Schutz durch die Neemformulierung hält außerdem höchstens 14 Tage an, dann sind die Wirkstoffe durch Umwelteinflüsse zerstört und das Feld muß wieder eingesprüht werden. Besser und effektiver können diese fraßhemmenden Formulierungen zum Schutz von gelagerten Erzeugnissen wie gelagertem Reis und Mais eingesetzt werden. Mit Hilfe eines Fraßtests an der Wüstenheuschrecke konnten Butterworth und Morgan 1986 die am stärksten fraßhemmende Substanz, die sie als Azadirachtin bezeichneten, aus Neemkernen isolieren. Es wurde außerdem festgestellt, daß Azadirachtin eine Wachstumsstörung bei verschiedenen holometabolen Insekten verursachte. Die Larvenentwicklung bis zum Addultstadium war vermindert und Mortalität wurde im Puppenstadium beobachtet. Da der Gewichtszuwachs der behandelten Tiere dem der Kontrolltiere entsprach, konnte eine Fraßhemmung ausgeschlossen werden. Die Wirkung von Azadirachtin auf die Larven-Puppen-und Puppen-Addult-Häutung wurde als eine Störung des Häutungshormonpools interpretiert. Die wachstumshemmende Wirkung bei Larven sowie die Hemmung der Eireifung bei erwachsenen Insekten wurde noch bei einer Vielzahl anderer Insekten beobachtet.

Da die bisher verwendeten Insektizide entweder für den Menschen oder die Umwelt schädlich sind oder aber nicht besonders stark wirksam sind, liegt der Erfindung die Aufgabe zugrunde, Verbindungen auf Azadirachtinbasis aufzufinden, welche die oben erwähnten Nachteile nicht oder nur in verringertem Maße aufweisen, insbesondere Verbindungen mit verstärkter Wirksamkeit und geringerer Schädlichkeit für andere Tiere und Menschen, sowie diese als Wirkstoff in Insektenvernichtungsmitteln einzusetzen. Weiter betrifft die Erfindung die Herstellung eines Insektenvernichtungsmittels aus einem Extrakt von Kernen der Nüsse des Neembaumes.

Gelöst wird diese Aufgabe durch die Verbindungen der allgemeinen Formel I,

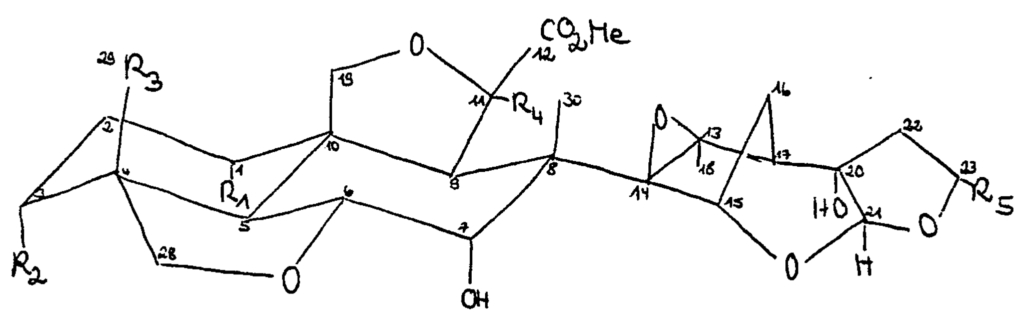

wobei

$R_1$ eine Hydroxylgruppe oder eine Acylgruppe einer geradkettigen oder verzweigten gesättigten, oder wenn $R_2$ eine andere Acylgruppe als Acetyl oder/und $R_4$ H oder/und $R_5$ H oder eine Alkoxygruppe ist, ungesättigten Monocarbonsäure mit bis zu 10 C-Atomen, wie z. B. Acetyl, Propionyl, n-Butyryl, Isobutyryl, n-Valeroyl, Iso-Valeroyl, n-Caproyl, Tigloyl

$R_2$ ebenso eine Hydroxylgruppe oder eine Acylgruppe einer geradkettigen oder verzweigten gesättigten oder, wenn $R_1$ eine Acylgruppe oder/und $R_4$ OH oder/und $R_5$ H oder eine Acylgruppe ist, ungesättigten Monocarbonsäure mit bis zu 10 C-Atomen, wie Acetyl, Propionyl, n-Butyryl, Isobutyryl, n-Valeroyl, Iso-Valeroyl, n-Caproyl, Tigloyl,

$R_3$ $CO_2CH_3$ oder Methyl,

$R_4$ H, Hydroxyl oder Acetyl und

$R_5$ H, eine Alkoxygruppe mit bis zu 6 C-Atomen (sowohl in alpha-als auch in beta-Stellung) oder eine zusätzliche Bindung zu C-22 sein kann. Die Verbindung der C-Atome 22 und 23 kann also sowohl eine C-C-Einfach-oder aber auch eine C-C-Doppelbindung sein.

Bevorzugt sind Verbindungen, bei denen $R_1$ und $R_2$ jeweils OH, Acetyl, oder unter den obengenannten Bedingungen Tigloyl, $R_4$ H oder Hydroxyl und $R_5$ H oder $OCH_2CH_3$ oder eine zusätzliche Bindung zu C-22 sind. Am meisten bevorzugt sind Verbindungen, bei denen $R_1$ und $R_2$ jeweils eine Hydroxylgruppe sind, wie z. B. beim 3-Detigloyl-azadirachtin B. Die Unteransprüche kennzeichnen weitere bevorzugte Verbindungen.

Diese Substanzen wurden entweder aus den Nüssen von Neembäumen isoliert oder aus Azadirachtin A oder B chcmisch durch Derivatisierung hergestellt. Die Strukturen dieser Substanzen wurden durch spektroskopische Methoden, hauptsächlich NMR, aufgeklärt.

Bei den Substanzen Deacetyl-azadirachtin A, Detigloylazadirachtin B und Azadirachtin F sind nur die Substituenten am Dekalinsystem unterschiedlich. Der Epoxidring an C-13-C-14, die Ketalfunktion an C-21, der Dihydrofuranring und die Hydroxylgruppe an C-7 sind bei allen drei Substanzen gleich und vermutlich für die Wirksamkeit von Bedeutung. In den Verbindungen, welche Gegenstand der Unteransprüche 4 bis 7 sind, ist die Furandoppelbindung derivatisiert. Gegenüber den Stammverbindungen Azadirachtin A und Azadirachtin B ist die Aktivität der Verbindungen am höchsten, wenn die Hydroxylgruppen an C-1 und C-3 nicht verestert sind. Ebenso haben alle Verbindungen, die an der Furandoppelbindung derivatisiert wurden, eine höhere Aktivität als die jeweilige Stammverbindung Azadirachtin A oder B. Vor allem die Aktivität der beiden Ethoxyverbindungen ist stark erhöht. In Tabelle 1 wird ein Aktivitätstest der verschiedenen Verbindungen im Vergleich zu Azadirachtin A und Azadirachtin B gezeigt.

Hierfür wurden verschiedene Konzentrationen jeder Substanz getestet. Jede Konzentration ergab einen Mortalitätswert. Aus diesen Mortalitätswerten wurden mit dem Computerprogramm von Noack und Reichmuth, Mitt. Biol. Bundesanst. Land-u. Forstwirtsch. Heft 185, August 1978, die lethale Konzentration für 50 % der Tiere ($LC_{50}$) berechnet. Die lethale Konzentration wurde dabei in ppm angegeben. Aus der Tabelle 1 ist ersichtlich, daß Detigloylazadirachtin B die höchste insektizide Aktivität der untersuchten Verbindungen aufweist. Jedoch liegen auch alle anderen getesteten Verbindungen in ihrer Aktivität zum Teil weit oberhalb der Aktivität der Stammverbindungen Azadirachtin A und B.

Das erfindungsgemäße Verfahren zur Herstellung eines Insektenvernichtungsmittels aus dem Rohextrakt von Kernen von Nüssen des Neembaumes ist dadurch gekennzeichnet, daß der ein natürliches Azadirachtingemisch enthaltende Extrakt unter so milden Bedingungen verseift wird, daß bei den vorhandenen natürlichen Azadirachtinen und ihren Derivaten eine vorhandene Estergruppe an C-1 oder/und eine vorhandene Estergruppe an C-3, auf keinen Fall jedoch die Epoxygruppe zwischen C-13 und C-14, hydrolysiert wird. Dies kann durch Verseifen in schwach basischer Lösung bei Raumtemperatur erreicht werden. Bevorzugt wird die Verseifung hierbei in wäßriger Lösung eines Alkali-oder Erdalkalicarbonats, beispielsweise in 10%iger $K_2CO_3$-Lösung durchgeführt. Dadurch werden alle Azadirachtine und ihre Derivate in die wirkungsstärkste Form, die analog dem 3-Detigloylazadirachtin B an C-1 und C-3 eine Hydroxylgruppe aufweisen, übergeführt. Andere, dem Fachmann bekannte vergleichbar milde Verseifungsmethoden lassen sich ebenfalls anwenden.

Erfindungsgemäße Insektenvernichtungsmittel sind dadurch gekennzeichnet, daß sie als Wirkstoff mindestens eine Verbindung, die als Strukturelement ein Azadirachtingerüst der allgemeinen Formel

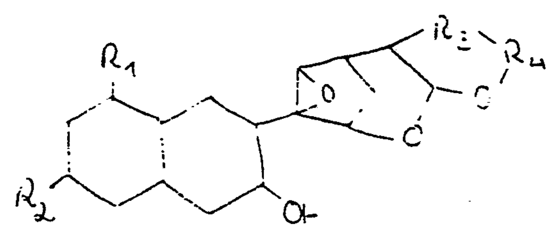

wobei

$R_1$ entweder Hydroxyl-oder eine Acylgruppe einer geradkettigen oder verzweigten gesättigten oder, wenn $R_2$ eine andere Acylgruppe als Acetyl oder/und $R_3$ sowie $R_4$ jeweils C mit H oder einer Alkoxygruppe als Ligand sind, ungesättigten Monocarbonsäure mit bis zu 10 C-Atomen, z. B. Acetyl, Propionyl, n-Butyryl, Isobutyryl, n-Valeroyl, Isovaleroyl, n-Capronyl, Tigloyl, $R_2$ entweder Hydroxyl oder eine Acylgruppe einer geradkettigen oder verzweigten gesättigten oder, wenn $R_1$ eine Acylgruppe oder/und $R_3$ sowie $R_4$ jeweils C mit H oder einer Alkoxygruppe als Ligand sind, ungesättigten Monocarbonsäure mit bis zu 10 C-Atomen, z. B. Acetyl, Propionyl, n-Butyryl, Isobutyryl, n-Valeroyl, Isovaleroyl, n-Capronyl, Tigloyl, und $R_3$ sowie $R_4$ jeweils ein C-Atom mit einem H oder einer Alkoxygruppe mit bis zu 6 C-Atomen als Liganden, wobei die CC-Bindung zwischen $R_3$ und $R_4$ entweder eine Einfach-oder eine Doppelbindung sein kann, sind, und

außerdem gegebenenfalls übliche Träger-oder/und Verdünnungsmittel enthalten. Bevorzugt sind hierbei Verbindungen, bei denen $R_1$ und $R_2$ jeweils OH, Acetyl oder, unter den obengenannten Bedingungen, Tigloyl, und $R_3$ und $R_4$ jeweils C mit H oder OEt als Liganden oder einer zusätzlichen Bindung zu $R_4$ bzw. $R_3$ ist. Besonders bevorzugt sind $R_1$ und $R_2$ beide OH. Andere erfindungsgemäße Insektenvernichtungsmittel enthalten mindestens eine der Verbindungen, welche Gegenstand der Ansprüche 1 bis 10 sind.

Durch die in den erfindungsgemäßen Insektenvernichtungsmitteln vorhandenen Substanzen wird die Entwicklung von Insekten wie am Beispiel des Bohnenkäfers untersucht wurde, vom $L_4$-Stadium bis zum erwachsenen Tier beeinträchtigt. Normalerweise durchläuft das Tier mehrere Entwicklungsstadien, wenn das Wachstum nicht gestört wird. Aus der Larve im vierten Stadium entwickelt sich eine Vorpuppe, die dann in das Puppenstadium übergeht. Aus der Puppe schlüpft das erwachsene Tier. Nach Gabe von Azadirachtin oder seinen Derivaten können in diesen Entwicklungsstadien verschiedene Störungen des Wachstums beobachtet werden:

a) Verlängerung des Larvenstadiums bis hin zur Dauerlarve,

b) hat sich eine Vorpuppe entwickelt, trocknet diese aus,

c) Austrocknen der normalen Puppe,

d) Entwicklung einer normalen Puppe, es gelingt dem Tier jedoch nicht, die Puppenhülle abzustreifen, es stirbt an Entkräftung;

e) ein erwachsenes Tier schlüpft aus der Hülle, hat jedoch derartige Deformationen, daß es nicht lebensfähig ist.

Das einzelne Tier erleidet nur eine dieser Wachstumsstörungen. Jede Störung reicht aber aus, um eine normale Entwicklung zu unterbinden. Neben der wachstumshemmenden Wirkung der erfindungsgemäßen Verbindungen ist außerdem eine Fraßhemmung möglich.

Die folgenden Beispiele sollen die Erfindung näher erläutern.

**Beispiel 1**

Isolierung von Azadirachtinen und anschließende Derivatisierung

Aufarbeitung von 57 kg Neemnüssen:

Die Neemnüsse wurden in einer Weizenentspelzungsanlage von den Schalen befreit. Die Trennung der Schalenteile und der Kerne erfolgte in einer Windsichtungsanlage. Die Schalen (30 kg) enthalten kein Azadirachtin. Sie wurden verworfen. Die weitere Aufarbeitung wurde mit Kernen (27 kg) fortgesetzt. Um für die anschließende Extraktion bessere Bedingungen zu schaffen, wurden die Neemkerne mit einer Getreidemühle gemahlen. Das ölige Pulver wurde möglichst schnell extrahiert, um enzymatische Abbaureaktionen zu vermeiden. Die 27 kg gemahlenen Neemkerne wurden in 5 Portionen aufgeteilt. Jede Portion wurde zweimal mit 10 l techn. Hexan zwei Stunden gerührt. Danach wurde filtriert und die Extrakte der einzelnen Portionen wurden vereinigt. Nach Abdestillieren des Lösungsmittels blieben 12,0 kg Neemöl zurück. Der Gasraum in dem 20 l-Kolben wurde ständig mit Luft gespült. Dadurch konnte kein zündfähiges Gemisch entstehen. 15 kg entöltes Neempulver blieben zurück. Dieses Pulver wurde in 3 Portionen geteilt und in der gleichen Apparatur je zweimal mit je 10 l techn. Aceton extrahiert. Die vereinigten Aceton-Trockenextrakte hatten ein Gewicht von 1670 g. Eine Analyse mit Dünnschicht-Chromatographie zeigte, daß Azadirachtin nur in der Acetonfraktion enthalten war. Die vereinigten Acetonfraktionen wurden in Methanol gelöst und mit 1500 g Kieselgel vermischt. Das Methanol wurde abdestilliert, die Acetonfraktion verblieb auf dem Kieselgel. Zur Filtration wurde eine Säule von 30 cm Länge und 10 cm Durchmesser mit Kieselgel gefüllt. Die Acetonfraktion (1,6 kg) auf Kieselgel wurde in 5 Portionen geteilt. Jede Portion wurde auf der obengenannten Kieselgelsäule mit zwei verschiedenen Petrolether/Essigester-Gemischen eluiert. Zuerst wurde mit 4 l Petrolether/ Essigester (7:3), dann mit 4 l Petrolether/Essigester (1:4) eluiert. Die Kieselgelsäule wurde danach mit Methanol regeneriert und konnte wieder verwendet werden. Die Hauptmenge, nämlich 1.140 g Azadirachtin war in den vereinigten ersten Fraktionen enthalten, eine kleinere Menge, nämlich 170 g, war in den vereinigten zweiten Fraktionen enthalten. Ein Teil dieser geringen Menge wurde durch Extraktion mit Methanol/Wasser/Hexan (4:1:5) extrahiert. Dabei enthielt die Methanolphase die Hauptmenge des Azadirachtin (etwa 95 %). Danach wurden die Azadirachtinverbindungen durch Säulenchromatographie über eine 300 mm lange Säule mit 25 mm Durchmesser, die mit 100 g $SiO_2$-RP-8-Material gefüllt war, abgetrennt. Das Laufmittel Methanol/Wasser (7:3) war für diese Zwecke am besten geeignet. Für jeden Lauf wurden 7 g der Methanolfraktion der zweiten Fraktionen aus dem vorhergehenden Reinigungsschritt eingesetzt. Fraktionen wurden von Beginn an entnommen.

Jede Fraktion entsprach 10 Minuten Elutionszeit (etwa 15 ml Lösungsmittel). Die Fraktionen wurden vom Lösungsmittel befreit, gewogen und mit Dünnschicht-Chromatographie analysiert. Fig. 1 zeigt ein Gewichtsdiagramm der Säulenchromatographie an RP-8-$SiO_2$. Auf der Abszisse sind die Fraktionen aufgetragen, auf der Ordinate die Trockengewichte der Fraktionen. Dieses Gewichtsdiagramm zeigt zwei Maxima. Das erste Maximum entspricht Azadirachtin A, das zweite Maximum nicht einem Azadirachtin B-Peak, sondern enthält eine andere unbekannte Verbindung. Azadirachtin B ist in den Fraktionen 6, 7 und 8 angereichert.

Die Fraktionen um die beiden Maxima wurden aus insgesamt 8 Portionen zusammengegeben. 15,5 g Azadirachtin A-Fraktion, sowie 8,3 g Azadirachtin B-Fraktion wurden erhalten. Die Azadirachtin A-Fraktion enthielt jedoch noch sehr viel Azadirachtin B, während die Azadirachtin B-Fraktion noch andere Substanzen in fast gleicher Menge wie Azadirachtin B enthielt. Um Azadirachtin A und B zu trennen, wurde präparative HPLC benutzt. Hierfür wurde eine LATEK-Säule, gefüllt mit Kieselgel-RP-18-Material mit einer Länge von 540 mm und einem Innendurchmesser von 54 mm benutzt. Die Trennung wurde opti miert. Die Durchflußrate betrug 55 ml/min mit Methanol/Wasser (43:57) als Laufmittel. Für jeden Lauf wurden etwa 5 g der Azadirachtin A-Fraktion der Säulenchromatographie an Kieselgel-RP-8 auf die Säule aufgetragen. Alle 9 Minuten wurde die Vorlage gewechselt bei einem Volumen von etwa 500 ml. Das Maximum von Azadirachtin A wurde nach 284 Minuten eluiert. Das Maximum von Azadirachtin B erschien nach 397 Minuten. Fig. 2 zeigt eine präparative HPLC-Chromatographie der Azadirachtin A-Fraktion bei Chromatographie an RP-8. Reines Azadirachtin A und Azadirachtin B war nur in den Fraktionen um das Peak-Maximum enthalten. Fünf weitere Azadirachtine wurden in Nebenfraktionen der präparativen HPLC mit Dünnschichtchromatographie oder halbpräparativer HPLC im kleineren Maßstab weiter aufgetrennt. Aus Azadirachtin A und Azadirachtin B wurden die verschiedenen Azadirachtin-Derivate hergestellt.

**Beispiel 2**

Isolierung von Azadirachtin F

Die Substanz wurde aus einer polaren Fraktion der präparativen HPLC isoliert. 20 mg dieser Fraktion wurden auf zwei 0,5 mm-20×20-cm-$SiO_2$-Platten aufgetragen und mit dem Laufmittel $CHCl_3$/Aceton (7:3) chromatographiert. Die Bande mit $R_f$ 0,27 wurde ausgekratzt. Ausbeute: 4,50 mg. Analytik: Laufmittel:Methanol/Wasser (2:3); Säule 100×5 mm; 3 µm-RP-18-Material. Azadirachtin F: RT 4,91 Minuten. (Azadirachtin A 8,8 Minuten).

**Beispiel 3**

Darstellung von 22,23-Dihydroazadirachtin A

40,8 mg Azadirachtin A ($5{,}67 \times 10^{5}$ Mol) wurden in 4 ml Essigester gelöst. Dazu wurden 20 mg $PtO_2 \times 2\ H_2O$ gegeben. Die Mischung wurde gerührt und 1 1/4 Stunden lang $H_2$ durchgeleitet. Der Verlauf der Reaktion wurde mit HPLC überwacht. Nach Verschwinden des Azadirachtin A-Peaks wurde die Reaktion unterbrochen und die Reaktionsmischung zentrifugiert. Die überstehende Lösung wurde eingeengt und auf 0,5 mm $SiO_2$-DC-Platten gereinigt. Laufmittel $CHCl_3$/Aceton (7:3),$R_f$ 0,33. Ausbeute: 28,2 mg (69% d.Th.)

**Beispiel 4**

Darstellung von 22,23-Dihydroazadirachtin B

95 mg Azadirachtin B ($1{,}44 \times 10^{4}$ Mol) wurden in 7 ml Essigester gelöst. Dazu wurden 45 mg $PtO_2 \times 2H_2O$ gegeben, gerührt und zwei Stunden lang $H_2$ durchgeleitet. Danach wurde die Mischung filtriert und zentrifugiert. Der Überstand wurde eingeengt und mit DC gereinigt. Laufmittel: Ether/Aceton (4:1), $R_f$ 0,37. Ausbeute: 68 mg (67 % d. Th.).

**Beispiel 5**

Darstellung von 23α-Ethoxy-22,23-dihydroazadirachtin A und 23β-Ethoxy-22,23-dihydroazadirachtin A

30 mg Azadirachtin A ($4{,}2 \times 10^{5}$ Mol) wurden in 0,5 ml $CHCl_3$ gelöst. Zu dieser Mischung wurden 120μl Acetylierungslösung (50μl Acetylchlorid in 2 ml $CHCl_3$ gegeben. Nach 10 Minuten wurde nicht umgesetztes Acetylchlorid mit Wasser zerstört. Der Ansatz wurde mit $NaHCO_3$-Lösung neutralisiert, dann mit $CHCl_3$ extrahiert. Die abschließende Reinigung erfolgte mit DC auf 0,5 mm-$SiO_2$-Platten. Laufmittel: $CHCl_3$/Aceton (7:3)
Es gab zwei Hauptkomponenten:
1. $R_f$ 0,54: 23α-Ethoxy-Derivat Ausbeute: 5,2 mg
2. $R_f$ 0,24: 23β-Ethoxy-Derivat Ausbeute: 2,4 mg

Beim Ansatz mit Benzoylchlorid anstelle von Acetylchlorid entstand nur das 23α-Ethoxy-Derivat. Der Umsatz war erst nach Stehen über Nacht vollständig.

**Beispiel 6**

Darstellung von 3-Deacetylazadirachtin A

15,8 mg Azadirachtin A ($2{,}2 \times 10^{5}$ Mol) wurden in 100μl Methanol gelöst. Zu dieser Lösung wurden dreimal jeweils 100 μl einer methanolischen Natriummethylat-Lösung gegeben (0,6 g Natrium in 50 ml Methanol). Nach jeder Zugabe von 100 μl Methylat-Lösung wurde 2 Minuten gewartet, dann mit HPLC überprüft, ob noch Azadirachtin A vorhanden war. Nach dem Verschwinden von Azadirachtin A wurde mit Essigsäure neutralisiert, mit $H_2O$ verdünnt und mit $CHCl_3$ extrahiert. Die Reinigung von den Nebenprodukten erfolgte mit der halbpräp. HPLC auf einer 20cm-RP-18-Säule mit 9 mm Innendurchmesser. Laufmittel: Methanol/Wasser (2:3), Detektion bei 215 nm, Ausbeute: 2,4 mg (15,2 %).

**Beispiel 7**

Darstellung von 3-Detigloylazadirachtin B

20 mg Azadirachtin B ($3{,}0 \times 10^{5}$ Mol) wurden in 1 ml Methanol gelöst. Dazu wurden 5 ml 10%ige wäßrige $K_2CO_3$-Lösung gegeben. Nach 12 Stunden wurde mit Chloroform extrahiert. Die Chloroform-Phase wurde eingeengt und mit DC gereinigt.
Laufmittel: $CHCl_3$/Aceton (7:3), $R_f$ 0,13
Ausbeute: 5,2 mg (26% d. Th.).

Tabelle 1

| Substanz | $LC_{50}$ (ppm) |
|---|---|
| Azadirachtin A | 1,66 |
| Azadirachtin B | 1,30 |
| Deacetylazadirachtin A | 0,38 |
| Detigloylazadirachtin B | 0,08 |
| Azadirachtin F | 1,15 |
| 23α-Ethoxy-22,23-dihydroaza A | 0,74 |
| 23ß-Ethoxy-22,23-dihydroaza A | 0,52 |
| 22,23-Dihydroazadirachtin A | 1,26 |
| 22,23-Dihydroazadirachtin B | 0,28 |

## Ansprüche

1. Verbindungen der allgemeinen Formel

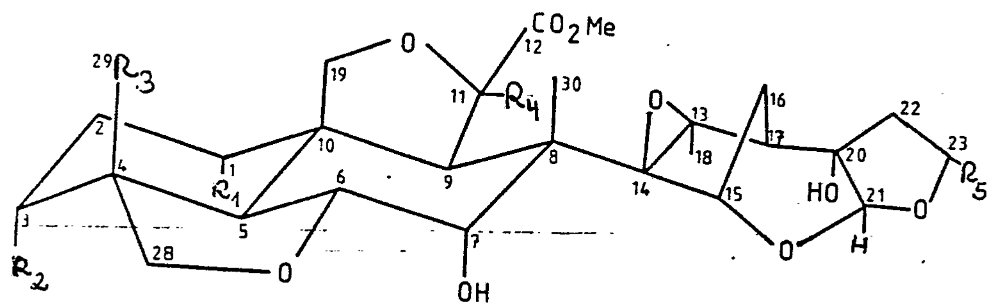

wobei

$R_1$ = OH oder eine Acylgruppe einer geradkettigen oder verzweigten gesättigten oder, wenn $R_2$ eine andere Acylgruppe als Acetyl oder/und $R_4$ H oder/und $R_5$ H oder eine Alkoxygruppe ist, ungesättigten Monocarbonsäure mit bis zu 10 C-Atomen,

$R_2$ = OH oder eine Acylgruppe einer geradkettigen oder verzweigten gesättigten oder, wenn $R_1$ eine Acylgruppe oder/und $R_4$ OH oder/und $R_5$ H oder eine Alkoxygruppe ist, ungesättigten Monocarbonsäure mit bis zu 10 C-Atomen,

$R_3$ = $CO_2CH_3$, $CH_3$;

$R_4$ = H, OH oder OAc;

$R_5$ = H, eine Alkoxygruppe mit bis zu 6 C-Atomen (sowohl in alpha-als auch in beta-Stellung), oder eine zusätzliche Bindung zu C-22;

bedeutet.

2. Verbindung nach Anspruch 1, **dadurch gekennzeichnet,** daß $R_1$ OH, Acetyl oder, wenn $R_2$ eine andere Acylgruppe als Acetyl oder/und $R_4$ H oder/und $R_5$ H oder eine Alkoxygruppe ist, Tigloyl, $R_2$ OH, Acetyl oder, wenn $R_1$ eine Acylgruppe oder/und $R_4$ OH oder/und $R_5$ H oder eine Alkoxygruppe ist, Tigloyl, $R_3$ $CO_2CH_3$ oder $CH_3$, $R_4$ H oder OH und $R_5$ H oder $OCH_2CH_3$ (sowohl in alpha-als auch in beta-Stellung) oder eine zusätzliche Bindung zu C-22 bedeuten.

3. Verbindungen nach Anspruch 1, **dadurch gekennzeichnet,** daß $R_1$ und $R_2$ OH bedeuten.

4. Azadirachtin F mit den Formel:

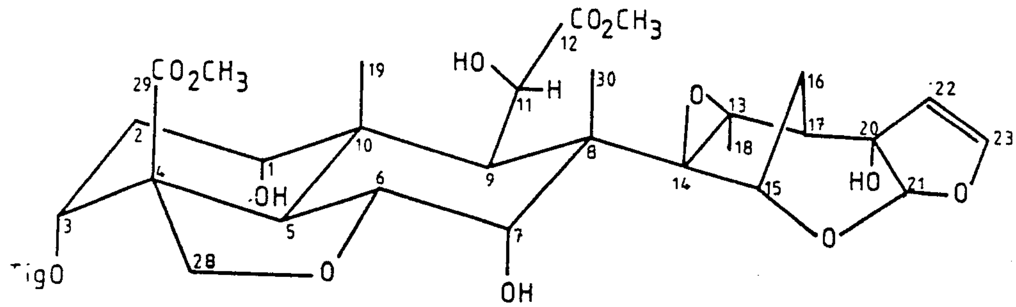

5. 22,23-Dihydro-azadirachtin A mit der Formel

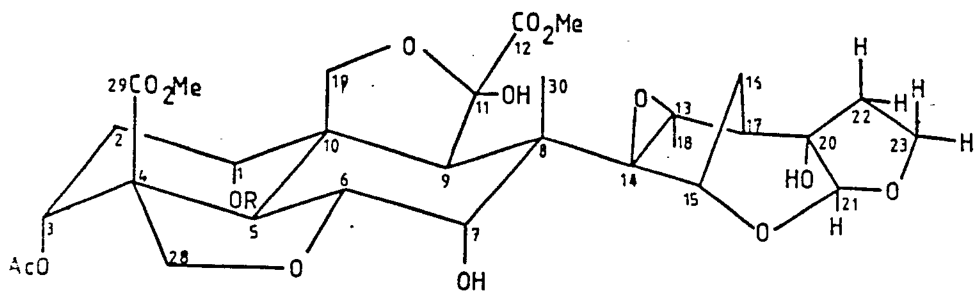

R = Tigloyl

6. 22,23-Dihydro-azadirachtin B mit der Formel

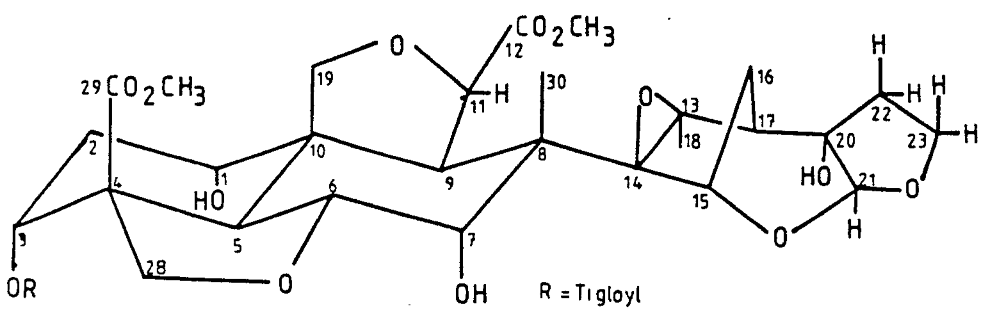

R = Tigloyl

7. 23-α-Ethoxy-22,23-dihydro-azadirachtin A mit der Formel

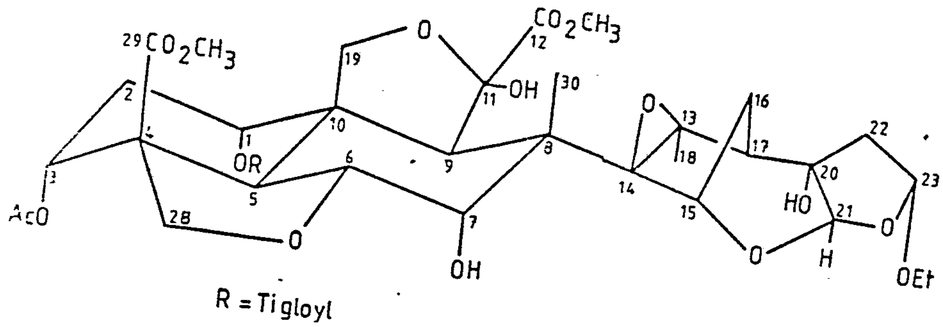

R = Tigloyl

8. 22-β-Ethoxy-22,23-dihydro-azadirachtin A mit der Formel

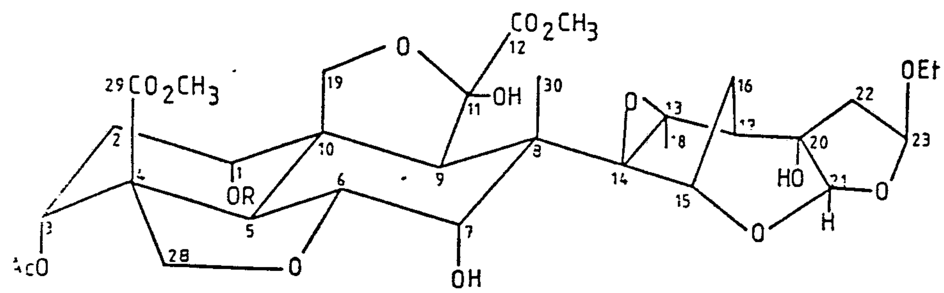

R = Tigloyl

9. 3-Deacetyl-azadirachtin A mit der Formel

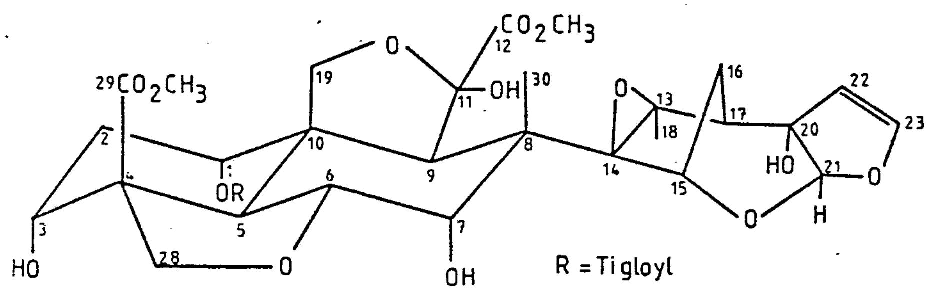

R = Tigloyl

10. 3-Detigloyl-azadirachtin B mit der Formel

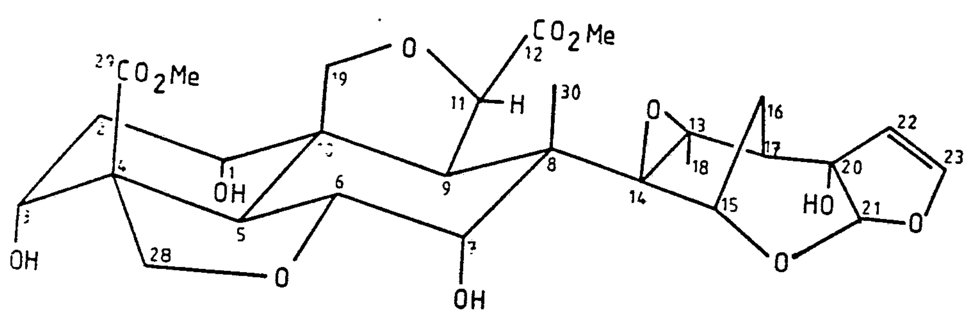

11. Insektenvernichtungsmittel, **dadurch gekennzeichnet,** daß es als Wirkstoff mindestens eine Verbindung, die als Strukturelement ein Azadirachtingerüst der allgemeinen Formel

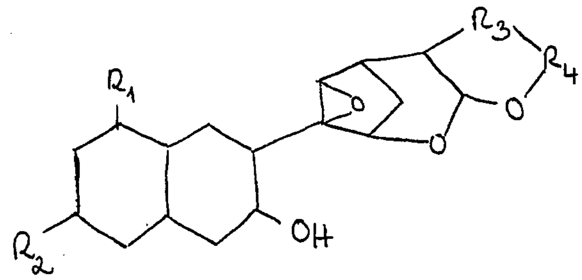

wobei

$R_1$ = OH oder ein Acylrest einer geradkettigen oder verzweigten gesättigten oder wenn $R_2$ eine andere Acylgruppe als Acetyl, oder/und $R_3$ sowie $R_4$ jeweils C mit H oder einer Alkoxygruppe als Ligand sind, ungesättigten Monocarbonsäure mit bis zu 10 C-Atomen,

$R_2$ = OH oder ein Acylrest einer geradkettigen oder verzweigten gesättigten oder, wenn $R_1$ eine Acylgruppe oder/und $R_3$ sowie $R_4$ jeweils C mit H oder einer Alkoxygruppe als Ligand sind, ungesättigten Monocarbonsäure mit bis zu 10 C-Atomen,

$R_3$ und $R_4$ jeweils C mit H oder einer Alkoxygruppe mit bis zu 6 C-Atomen als Liganden oder einer zusätzlichen Bindung zu $R_4$ bzw. $R_3$, bedeutet,

und gegebenenfalls übliche Träger-oder/und Verdünnungsmittel enthält.

12. Insektenvernichtungsmittel nach Anspruch 11, **dadurch gekennzeichnet,** daß

$R_1$ OH, Acetyl oder, wenn $R_2$ eine andere Acylgruppe als Acetyl, oder/und $R_3$ sowie $R_4$ jeweils C mit H oder einer Alkoxygruppe als Ligand sind, Tigloyl,

$R_2$ OH oder Acetyl, oder wenn $R_1$ eine Acylgruppe oder/und $R_3$ sowie $R_4$ jeweils C mit H oder einer Alkoxygruppe als Ligand sind, Tigloyl,

$R_3$ und $R_4$ jeweils C mit H oder OEt als Liganden oder einer zusätzlichen Bindung zu $R_4$ bzw. $R_3$ bedeuten.

13. Insektenvernichtungsmittel nach Anspruch 11 oder 12, **dadurch gekennzeichnet,** daß $R_1 = R_2 =$ OH bedeuten.

14. Verfahren zur Herstellung eines Insektenvernichtungsmittels nach Anspruch 11 bis 13, **dadurch gekennzeichnet,** daß man einen Extrakt aus Kernen von Nüssen des Neembaumes in schwach basischer Lösung bei Raumtemperatur verseift.

15. Verfahren nach Anspruch 14, **dadurch gekennzeichnet,** daß die Verseifung in wäßriger Lösung eines Alkali-oder Erdalkalicarbonats durchgeführt wird.

16. Verfahren nach Anspruch 14 und 15, **dadurch gekennzeichnet,** daß die Verseifung in wäßriger $K_2CO_3$-Lösung durchgeführt wird.

17. Insektenvernichtungsmittel nach Anspruch 11, **dadurch gekennzeichnet,** daß es mindestens eine der Verbindungen nach den Ansprüchen 1 bis 10 enthält.

FIG.1

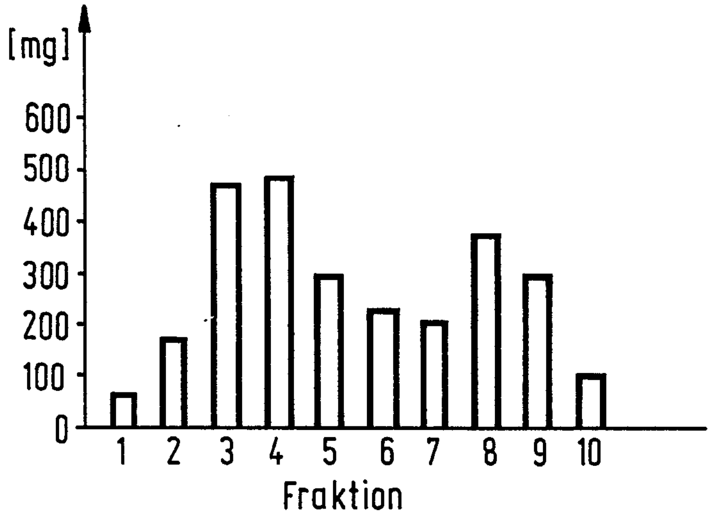


Gewichtsdiagramm der Säulenchromatographie an RP-8-$SiO_2$. Auf der Abszisse sind die Fraktionen aufgetragen, auf der Ordinate die Trockengewichte der Fraktionen

FIG.2

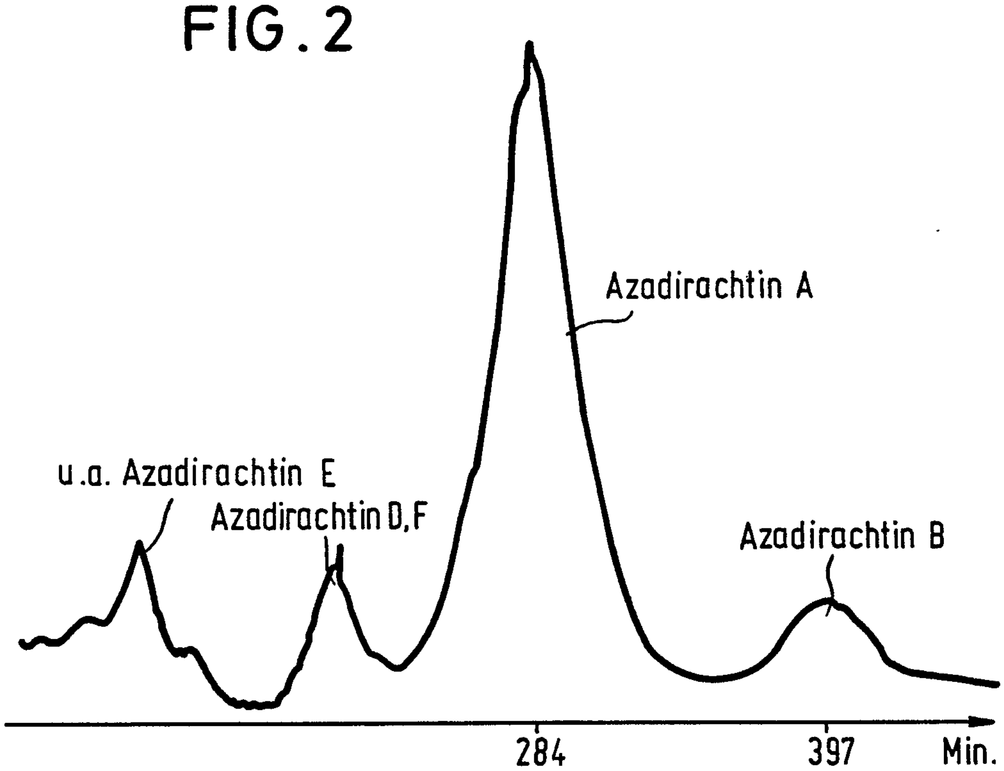


Präparative HPLC-Chromatographie der Azadirachtin A-Fraktion bei Chromatographie an RP-8. UV-Absorption bei 230 nm